# EUROPEAN PATENT SPECIFICATION

(11) **EP 3 972 493 B1**
(45) Date of publication and mention of the grant of the patent: **16.10.2024**
(21) Application number: 20726964.8
(22) Date of filing: 15.05.2020
(51) Int. Cl.: A61B 5/145, C12Q 1/00, G01N 27/327, G01N 33/487

(54) **COMPENSATION SYSTEM AND METHOD FOR THERMISTOR SENSING IN AN ANALYTE BIOSENSOR**
KOMPENSATIONSSYSTEM UND VERFAHREN ZUR THERMISTORERFASSUNG BEI EINEM ANALYT-BIOSENSOR
SYSTÈME DE COMPENSATION ET PROCÉDÉ DE DÉTECTION DE THERMISTANCE DANS UN BIOCAPTEUR D'ANALYTE

(30) Priority: 21.05.2019 US 201962850841 P
(43) Date of publication of application: 30.03.2022
(73) Proprietor: Ascensia Diabetes Care Holdings AG, 4052 Basel (CH)
(72) Inventor: HARRISON, Bern, Everett, Moscow, Idaho 83843 (US)
(74) Representative: Gille Hrabal Partnerschaftsgesellschaft mbB Patentanwälte
(86) International application number: PCT/IB2020/054643
(87) International publication number: WO 2020/234728

(56) References cited:
- WO-A1-2005/108968
- US-A1- 2009 177 406
- US-A1- 2011 203 942
- US-A1- 2012 095 312
- US-A1- 2018 188 199

## Description

### CROSS-REFERENCE TO RELATED APPLICATION

This application claims priority to and benefit of U.S. Provisional Patent Application No. 62/850,841, filed May 21, 2019.

### TECHNICAL FIELD

The present invention relates generally to a biosensor for analyte concentration (e.g., blood glucose) and more specifically a system that detects test sensor malfunctions in providing a temperature value from either an estimate temperature or a measured temperature in the process of analyte concentration determination.

### BACKGROUND

The quantitative determination of analytes in body fluids is of great importance in the diagnoses and maintenance of certain physiological conditions. For example, persons with diabetes (PWDs) frequently check the glucose level in their bodily fluids. The results of such tests can be used to regulate the glucose intake in their diets and/or to determine whether insulin or other medication needs to be administered. A PWD typically uses a measurement device (e.g., a blood glucose meter) that calculates the glucose concentration in a fluid sample from the PWD, where the fluid sample is collected on a test sensor that is received by the measurement device. The failure to take corrective action may have serious medical implications for that person.

One method of monitoring the blood glucose level of a PWD is with a portable testing device. The portable nature of these devices enables users to conveniently test their blood glucose levels at any location. One type of device utilizes an electrochemical test sensor to analyze the blood sample. A user employs a lancet to obtain a blood sample for application to a reservoir in the test sensor. The electrochemical test sensor typically includes electrodes that, when mated with the meter, electrically measures the reaction of the blood sample to determine an analyte concentration. A special meter device must therefore be carried by the user to determine the blood sample analysis.

Typically, a meter applies an input signal (e.g., a gated amperometry signal) to the electrodes of the test sensor. Traditional test sensors and meters typically use a glucose concentration estimation algorithm that determines the correlation between the measured current output from the blood sample and pre-determined analyte concentration values correlated with such outputs. These pre-determined values are determined by a laboratory instrument such as a YSI laboratory instrument.

The chemical reactions employed in the test sensors of any amperometric blood glucose monitoring (BGM) system are affected by temperature. A measured temperature value is therefore an important input into the glucose estimation algorithm of such systems. In known systems, temperature is measured by a thermistor based temperature sensors. The thermistor for the temperature sensor is typically located within the meter. Due to the thermal mass of the meter, thermistors cannot respond instantaneously to changes in the ambient temperature and thus distortions in temperature measurements can occur. When BGM meters are moved from one environment to another, a period of time is required for the meter to equilibrate to its new environment, and during this time the thermistor value will not accurately reflect the actual temperature. In the complex glucose estimation algorithms employed in gated amperometry meters, the temperature is included in many terms in the various compensation equations, and thus when the thermistor based temperature value is incorrect, erroneous results may occur.

Temperature estimates from thermistors are therefore subject to a risk that the meter has not equilibrated to its environment, resulting in erroneous temperature values being used by the algorithm. This creates a risk of inaccurate glucose readings. One solution for a non-equilibrated environment is to use an estimate temperature based on other parameters not derived from the thermistor. However, using the estimated temperature creates another risk from damaged sensors that may produce erroneous temperature estimates. Therefore, a large difference between the estimated temperature and the thermistor can indicate a damaged sensor in addition to a dis-equilibrated meter. In the case of a damaged sensor, the correct response is to report an error code. However, if the damaged sensor is not detected, the meter may attempt to calculate glucose using the temperature measurement from the damaged sensor, possibly resulting in inaccurate glucose readings.

Thus, there exists a need for a procedure to address the risk of inaccurate glucose results caused by a disequilibrated meter that relies solely on thermistor based temperature measurement. There is another need for a system that compares the estimated temperature to the measured temperature to provide information about whether the meter is properly equilibrated. There is another need for a system that allows use of an estimated temperature from a temperature estimation algorithm to determine analyte concentration even when disequilibration is detected. There is another need for a system that compares the estimated temperature to the measured temperature to provide information about whether the test sensor is damaged.

A biosensor with temperature compensation is known from US 2011/203942 A1.

### SUMMARY

According to independent claim 1, an analyte concentration sensor system for measuring an analyte of a fluid sample of a user is provided.

In another example, a method to determine suitability of a temperature measurement from a thermistor temperature sensor in an analyte meter, the analyte meter including a biosensor interface operable to be connected to a test sensor holding a fluid sample, and a controller, is set out in independent claim 13.

An input signal is generated to the interface when connected to the test sensor with the fluid sample. An output signal is determined from the test sensor. A measured temperature is determined from a thermistor based temperature sensor. An estimated temperature is determined from a temperature estimation algorithm via the controller. The difference between the estimated temperature and the measured temperature is determined via the controller. One of the estimated temperature or the measured temperature is selected via the controller based on the estimated temperature, the measured temperature, and the difference between the estimated temperature and the measured temperature. The selected estimated temperature or measured temperature is provided as a temperature input to an analyte concentration determination algorithm.

Another example is an analyte concentration sensor system for measuring an analyte of a fluid sample of a user. The sensor system includes a biosensor interface operable to be connected to a test sensor holding the fluid sample. The system includes a thermistor based temperature sensor configured to measure temperature. The system includes a controller coupled to the biosensor interface and the temperature sensor. The controller is operable to generate an input signal to the biosensor interface and read an output signal from the biosensor interface. The controller is operable to determine a measured temperature from the temperature sensor and execute a temperature estimation algorithm to determine an estimated temperature. The controller determines an absolute difference between the estimated temperature and the measured temperature. The controller determines a malfunction of the test sensor based on the estimated temperature, the measured temperature, and the absolute difference between the estimated temperature and the measured temperature.

Another example is a method to determine failure of a test sensor connected to an analyte meter. The analyte meter includes a biosensor interface operable to be connected to the test sensor holding a fluid sample, and a controller. An input signal is generated to the interface when connected to the test sensor with the fluid sample. An output signal is determined from the test sensor. A measured temperature is determined from a thermistor based temperature sensor. An estimated temperature is determined from a temperature estimation algorithm via the controller. An absolute difference between the estimated temperature and the measured temperature is determined via the controller. A malfunction of the test sensor is determined based on the estimated temperature, the measured temperature, and the absolute difference between the estimated temperature and the measured temperature.

Additional aspects of the invention will be apparent to those of ordinary skill in the art in view of the detailed description of various embodiments, which is made with reference to the drawings, a brief description of which is provided below.

### BRIEF DESCRIPTION OF THE DRAWINGS

FIG. 1 is a block diagram of an example biosensor system for determining analyte concentrations from a fluid sample according to one embodiment;
FIGs. 2A-2B are a flow diagram of the routine executed to select the temperature value to be used in the analyte concentration estimation algorithm executed by the biosensor in FIG. 1 according to one embodiment;
FIG. 3 is a state diagram of the responses of the routine in FIGs. 2A-2B showing the states of using the measured temperature, using the estimated temperature, or returning an error message;
FIG. 4 is a table of the parameters of one example of an estimation algorithm for temperature;
FIG. 5A is a summary table of the outputs of the temperature estimation algorithm in comparison to temperature measured by a thermistor based sensor;
FIG. 5B is a summary table of the accuracy of the outputs of the analyte concentration estimation algorithm using temperatures measured by a thermistor based sensor and outputs of the temperature estimation algorithm for studies conducted with meters in an equilibrated state;
FIG. 6 is a graph of an example input signal sequence for the temperature estimation algorithm;
FIG. 7A is a graph of the plots of the error of the outputs of the analyte concentration estimation algorithm using measured temperatures from tests conducted with meters in a wide range of equilibration states;
FIG. 7B is a graph of the plots of the error of the outputs of the analyte concentration estimation algorithm using estimated temperatures from tests conducted with meters in a wide range of equilibration states; and
FIG. 8 is a graph of the plots of the error of the outputs of the analyte concentration estimation algorithm using the final selected temperature (either estimated temperature or measured temperature) from tests conducted with meters in a wide range of equilibration states; and
FIG. 9 is a summary table of the accuracy of the outputs of the analyte concentration estimation algorithm for tests conducted with meters in three equilibration states (cold, hot, and equilibrated) comparing results calculated with temperature measured by a thermistor based sensor, temperature estimated with an algorithm using signals from the test sensor, and temperature chosen by logic based on the difference between the thermistor and the estimated temperature.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the spirit and scope of the invention as defined by the appended claims.

### DETAILED DESCRIPTION

The present disclosure is directed toward an analyte concentration measurement system that employs temperature equilibration logic to estimate ambient temperature using non-thermistor signals. The differential between the estimated temperature and the temperature measured from the thermistor is compared to defined thresholds to determine one of three actions to perform. The three actions include: 1) calculating the analyte concentration normally using the thermistor signal under the assumption that the meter is equilibrated and the test sensor signals are valid; 2) calculating the analyte concentration using an estimated temperature under the assumption that the meter is not equilibrated to the ambient conditions and the estimated temperature will produce a more accurate result; or 3) reporting an error under the assumption that the test sensor is compromised and the signals are not valid.

The logic that governs the three possible actions is as follows. When the estimated temperature and the thermistor measured temperature are in good agreement, both results are assumed to be accurate, and the thermistor measured temperature is used to calculate the analyte concentration because it produces the most reliable value under normal circumstances. When a large discrepancy is observed between the thermistor and the estimated temperature, there are two possible explanations: 1) the thermistor measured temperature is inaccurate because the meter is not equilibrated to the ambient environment; or 2) the estimated temperature is inaccurate because the sensor signals used to calculate the sensor were incorrect, either due to a damaged sensor or to perturbation of the sample during the test. The determination of whether to report a corrected result or an error message is based on an understanding of the most likely relationship between the thermistor measured temperature and the estimated temperature in each of the two possible scenarios described above. Since most glucose concentration testing occurs at room temperature, when the thermistor measured temperature is at an extreme value but the estimated temperature is normal, disequilibration is the most likely explanation, and the estimated temperature is used to calculate the analyte concentration. When the temperature reading from the thermistor is normal but the estimated temperature is extreme, it is more likely that the signal output waveform from the test sensor is anomalous and that an error should be reported.

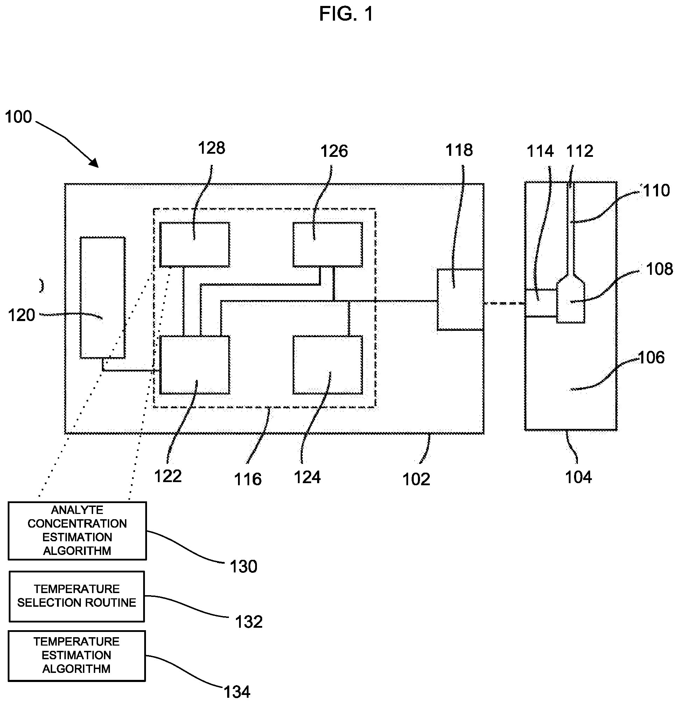

FIG. 1 depicts a schematic representation of a biosensor system 100 that determines an analyte concentration in a sample of a biological fluid. Biosensor system 100 includes a measurement device 102 and a test sensor 104, which may be implemented in any analytical instrument, including a bench-top device, a portable or hand-held device, or the like. The measurement device 102 and the test sensor 104 may be adapted to implement an electrochemical sensor system, an optical sensor system, a combination thereof, or the like. The biosensor system 100 determines analyte concentrations from output signals with a glucose estimation algorithm that uses a temperature input to correct its output for temperature. A temperature selection routine determines whether to use temperature measured from a thermistor sensor, an estimated temperature or return an error message. As will be explained, the routine improves the measurement performance of the biosensor system 100 in determining the analyte concentration of the sample by providing a more accurate temperature input into the analyte concentration estimation algorithm.

The biosensor system 100 may be utilized to determine analyte concentrations, including those of glucose, lipid profiles (e.g., cholesterol, triglycerides, LDL and HDL), microalbumin, hemoglobin A1_{C}, fructose, lactate, or bilirubin. It is contemplated that other analyte concentrations may also be determined. It is also contemplated that more than one analyte may be determined. The analytes may be in, for example, a whole blood sample, a blood serum sample, a blood plasma sample, other body fluids like urine, and non-body fluids. Thus, one example of the analyte concentration estimation algorithm is a glucose concentration estimation algorithm executed by the biosensor system 100. As used within this application, the term "concentration" refers to an analyte concentration, activity (e.g., enzymes and electrolytes), titers (e.g., antibodies), or any other measure concentration used to measure the desired analyte. While a particular configuration is shown, the biosensor system 100 may have other configurations, including those with additional components.

The test sensor 104 has a base 106 that forms a reservoir 108 and a channel 110 with an opening 112. The reservoir 108 and the channel 110 may be covered by a lid with a vent. The reservoir 108 defines a partially-enclosed volume. The reservoir 108 may contain a composition that assists in retaining a liquid sample such as water-swellable polymers or porous polymer matrices. Reagents may be deposited in the reservoir 108 and/or the channel 110. The reagents may include one or more enzymes, binders, mediators, and like species. The reagents may include a chemical indicator for an optical system. The test sensor 104 also may have a sample interface 114 disposed adjacent to the reservoir 108. The sample interface 114 may partially or completely surround the reservoir 108. The test sensor 104 may have other configurations.

In an optical sensor system, the sample interface 114 has an optical portal or aperture for viewing the sample. The optical portal may be covered by an essentially transparent material. The sample interface may have optical portals on opposite sides of the reservoir 108.

In an electrochemical system, the sample interface 114 has conductors connected to a working electrode and a counter electrode. The electrodes may be substantially in the same plane or in different planes. The electrodes may be disposed on a surface of the base 106 that forms the reservoir 108. The electrodes may extend or project into the reservoir 108. A dielectric layer may partially cover the conductors and/or the electrodes. The sample interface 114 may have other electrodes and conductors.

The measurement device 102 includes electrical circuitry 116 connected to a sensor interface 118 and a display 120. The electrical circuitry 116 includes a processor 122 connected to a signal generator 124, a temperature sensor 126, and a storage medium 128. In this example, the temperature sensor 126 operates by providing an electrical signal to a thermistor and reading an output signal from the thermistor that is proportional to the ambient temperature.

The signal generator 124 provides an electrical input signal to the sensor interface 118 in response to the processor 122. In optical systems, the electrical input signal may be used to operate or control the detector and light source in the sensor interface 118. In electrochemical systems, the electrical input signal may be transmitted by the sensor interface 118 to the sample interface 114 to apply the electrical input signal to the sample of the biological fluid. The electrical input signal may be a potential or current and may be constant, variable, or a combination thereof, such as when an AC signal is applied with a DC signal offset. The electrical input signal may be applied as a single pulse or in multiple pulses, sequences, or cycles, such as a gated ampometric signal. The signal generator 124 also may record an output signal from the sensor interface as a generator-recorder.

The temperature sensor 126 determines the temperature of the sample in the reservoir of the test sensor 104 based on the output signal of a thermistor in the sensor 126. As will be explained below, the temperature of the sample may be estimated by calculation from non-temperature signals, such as the output signal or signals, time, ratio between signals, and the like. The estimated temperature is assumed to be the same or similar to a measurement of the ambient temperature or the temperature of a device implementing the biosensor system. The temperature may be measured using another temperature sensing device.

The storage medium 128 may be a magnetic, optical, or semiconductor memory, another storage device, or the like. The storage medium 128 may be a fixed memory device, a removable memory device, such as a memory card, remotely accessed, or the like.

The processor 122 implements the analyte analysis and data treatment using computer readable software code and data stored in the storage medium 128. The processor 122 may start the analyte analysis in response to the presence of the test sensor 104 at the sensor interface 118, the application of a sample to the test sensor 104, in response to user input, or the like. The processor 122 directs the signal generator 124 to provide the electrical input signal or signals to the sensor interface 118. The processor 122 receives an output signal associated with the sample temperature from the temperature sensor 126. The processor 122 receives the output signal or signals from the sensor interface 118. The output signal is generated in response to the reaction of the analyte in the sample. The output signal may be generated using an optical system, an electrochemical system, or the like. The processor 122 determines compensated analyte concentrations from output signals using a glucose estimation algorithm as previously discussed. The results of the analyte analysis may be output to the display 120 and may be stored in the storage medium 128.

The correlation equations between analyte concentrations and output signals may be represented graphically, mathematically, a combination thereof, or the like. A correlation equation may include one or more index functions. Correlation equations may be represented by a program number (PNA) table, another look-up table, or the like that is stored in the storage medium 128. Constants and weighing coefficients also may be stored in the storage medium 128. Instructions regarding implementation of the analyte analysis may be provided by the computer readable software code stored in the storage medium 128. The code may be object code or any other code describing or controlling the functionality described herein. The data from the analyte analysis may be subjected to one or more data treatments, including the determination of decay rates, K constants, ratios, functions, and the like in the processor 122. In this example, the storage medium 128 stores an analyte concentration estimation algorithm 130 that determines the analyte concentration from inputs such as the signals from the sensor interface 118. The storage medium also stores a temperature selection routine 132 that determines a temperature value to input to the analyte concentration estimation algorithm 130. The storage medium 128 also stores a temperature estimation algorithm 134 for determining an estimated temperature value for the temperature selection routine 132.

In electrochemical systems, the sensor interface 118 has contacts that connect or electrically communicate with the conductors in the sample interface 114 of the test sensor 104. The sensor interface 118 transmits the electrical input signal from the signal generator 124 through the contacts to the connectors in the sample interface 114. The sensor interface 118 also transmits the output signal from the sample through the contacts to the processor 122 and/or signal generator 124.

In light-absorption and light-generated optical systems, the sensor interface 118 includes a detector that collects and measures light. The detector receives light from the liquid sensor through the optical portal in the sample interface 114. In a light-absorption optical system, the sensor interface 118 also includes a light source such as a laser, a light emitting diode, or the like. The incident beam may have a wavelength selected for absorption by the reaction product. The sensor interface 118 directs an incident beam from the light source through the optical portal in the sample interface 114. The detector may be positioned at an angle such as 45° to the optical portal to receive the light reflected back from the sample. The detector may be positioned adjacent to an optical portal on the other side of the sample from the light source to receive light transmitted through the sample. The detector may be positioned in another location to receive reflected and/or transmitted light.

The display 120 may be analog or digital. The display 120 may include a LCD, a LED, an OLED, a vacuum fluorescent, or other display adapted to show a numerical reading. Other displays may be used. The display 120 electrically communicates with the processor 122. The display 120 may be separate from the measurement device 102, such as when in wireless communication with the processor 122. Alternatively, the display 120 may be removed from the measurement device 102, such as when the measurement device 102 electrically communicates with a remote computing device, medication dosing pump, and the like.

In use, a liquid sample for analysis is transferred into the reservoir 108 by introducing the liquid to the opening 112. The liquid sample flows through the channel 110, filling the reservoir 108 while expelling the previously contained air. The liquid sample chemically reacts with the reagents deposited in the channel 110 and/or reservoir 108.

The test sensor 104 is disposed adjacent to the measurement device 102. Adjacent includes positions where the sample interface 114 is in electrical and/or optical communication with the sensor interface 118. Electrical communication includes the transfer of input and/or output signals between contacts in the sensor interface 118 and conductors in the sample interface 114. Optical communication includes the transfer of light between an optical portal in the sample interface 114 and a detector in the sensor interface 118. Optical communication also includes the transfer of light between an optical portal in the sample interface 114 and a light source in the sensor interface 118.

The processor 122 receives the measured temperature from the temperature sensor 126. The processor 122 directs the signal generator 124 to provide an input signal to the sensor interface 118. In an optical system, the sensor interface 118 operates the detector and light source in response to the input signal. In an electrochemical system, the sensor interface 118 provides the input signal to the sample through the sample interface 114. The processor 122 receives the output signal generated in response to the redox reaction of the analyte in the sample as previously discussed.

The processor 122 determines the analyte concentration of the sample via the analyte concentration estimation algorithm 130 in this example. One of the inputs to the analyte concentration estimation algorithm 130 is temperature, which is used to correct for the effect of differences in temperature on the sensor output signals.

The processor 122 in this example is operable to execute the temperature selection routine 132 that selects the temperature for the analyte concentration estimation algorithm 130. The processor 122 also executes the temperature estimation algorithm 134 that is capable of estimating the ambient temperature with sufficient accuracy that it can reliably detect disequilibration and be used by the analyte concentration estimation algorithm 130 to calculate an accurate result. The temperature selection routine 132 also includes logic to determine when a large discrepancy between the estimated temperature and the temperature measured by the thermistor of the temperature sensor 126 is caused by a damaged sensor rather than disequilibration, allowing an error code to be displayed on the display 122 rather than an incorrect result from executing the analyte concentration estimation algorithm 130 with the temperature output by a damaged temperature sensor. Alternatively, an error code can refer to an error index number or to an actual message displayed on the display 122 and/or recorded in memory.

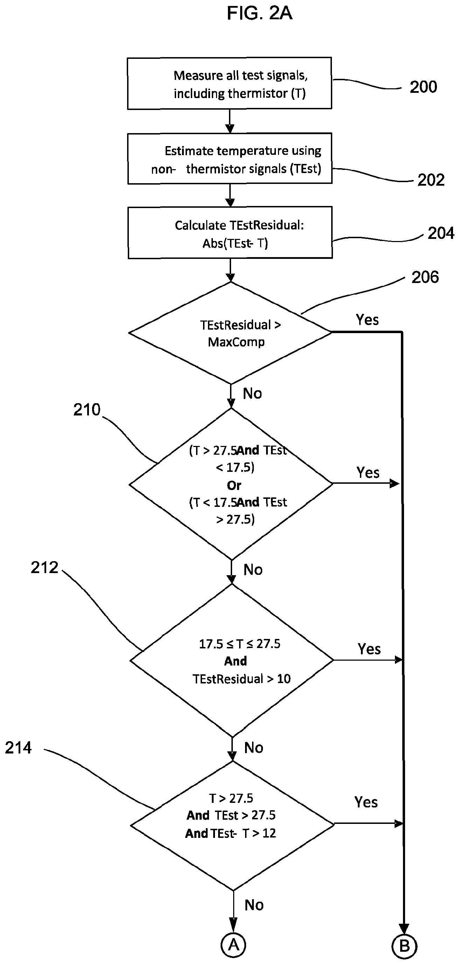

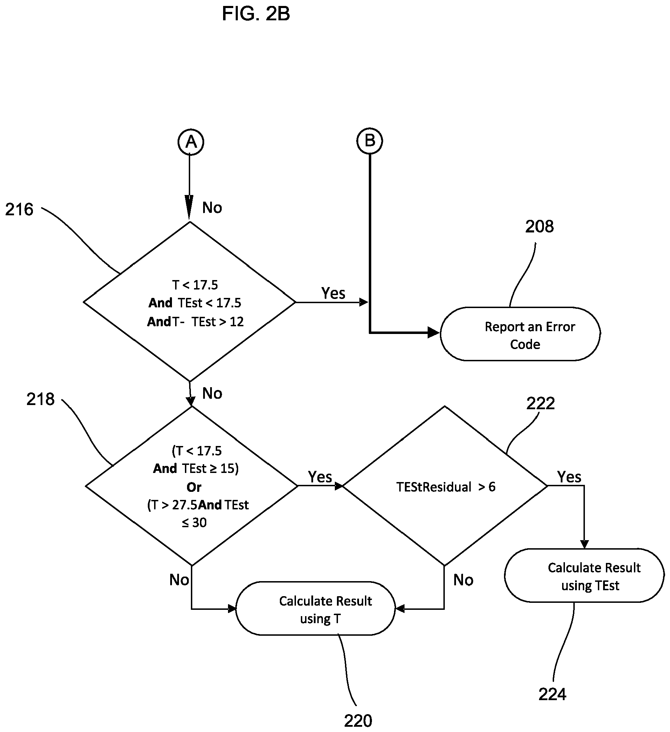

FIGs. 2A-2B are a flow diagram showing the temperature selection routine 132 in FIG. 1 that determines the temperature value to input into the analyte concentration estimation algorithm 130 in the example biosensor system 100. The routine 132 is executed on a controller such as the processor 122 in FIG. 1. Although it is better to use the estimated temperature when the thermistor is shifted by more than 3° C, it is impossible to know with certainty when this situation has actually occurred. The only information available to the temperature selection routine 132 is the difference between the thermistor and the estimated temperature. The temperature selection routine 132 in FIGs. 2A-2B therefore follows certain temperature compensation logic to determine whether to use the estimated temperature, the temperature from the thermistor, or return an error message.

The routine 132 first measures all the test signals (200). This includes applying an input signal from the signal generator 124 to the electrodes in the test sensor 104. The processor 122 reads the output signal from the biosensor interface 118. The test signal measurement step also includes the processor 122 reading a signal from the temperature sensor 126 in FIG. 1 and determining the measured ambient temperature (T). The processor 122 estimates the ambient temperature (T Est) based on the output signals read from the biosensor interface 118 and other inputs required by the temperature estimation algorithm 134 (202). The processor 122 then determines the absolute value of the difference between the estimated ambient temperature and the temperature measured by the temperature sensor 126 (T Est Residual) (204).

The processor 122 then determines whether the absolute value of the difference between the estimated ambient temperature and the temperature measured by the temperature sensor 126 exceeds the largest allowed temperature compensation value (MaxComp) (206). If the difference exceeds the largest allowed temperature compensation, the processor 122 reports an error code for the test sensor 104 (208). In this example, the largest variation between the estimated temperature and the temperature measured from the sensor 126 is 22° C, but other values may be used such as between 15° C to 25° C. Such a large difference indicates that the system 100 is unlikely to be in true disequilibrium and thus it is safer to report an error code that the test sensor 104 is malfunctioning.

If the difference is under the largest allowed temperature compensation value, the processor 122 then determines whether the measured temperature from the temperature sensor 126 and the estimated temperature are both within a room temperature range (210). In this example, the room temperature range is between 17.5° C and 27.5° C (e.g., 22.5 ±5°C), but other range values may be used. For example, the room temperature range could be defined differently depending on the expected typical use environment. Thus the high temperature of the room temperature range may be between 25°C and 30° C and the low temperature may be between 13° C and 20° C. If both the estimated temperature and the measured temperature are outside of the room temperature range in opposite direction, the processor 122 then reports an error code for the test sensor 104 (208).

If the temperature and the estimated temperature are both within the room temperature range, the processor 122 determines whether the measured temperature is within the room temperature range and determines if the difference between the measured temperature and estimated temperature is greater than a residual error limit threshold value (212). If the measured temperature is within the room temperature range, but the difference is greater than the residual error limit threshold value, the processor 122 reports an error code for the test sensor 104 (208). In this example, the residual error limit threshold value is 10° C, and differences above 10° C are an indication of a damaged test sensor, so an error code is reported. Depending on the system, the range of the residual error limit threshold value may be between 7° C and 15° C.

If the difference is less than the residual error limit threshold value, the processor 122 determines whether both the temperature and the estimated temperature are greater than the highest temperature of the room temperature range, and whether the difference between the estimated temperature and the measured temperature are greater than an extreme residual error limit threshold value (214). If these conditions are met, the processor 122 reports an error code for the test sensor 104 (208). In this example, the highest temperature of the room temperature range is 27.5° C and the extreme residual error limit threshold value is 12° C.

If these conditions are not met, the processor 122 determines whether both the measured temperature and the estimated temperature are less than the lowest temperature of the room temperature range, and whether the difference between the measured temperature and the estimated temperature is greater than the extreme residual error limit threshold value (216). In this example, the lowest temperature of the room temperature range is 17.5° C and the extreme residual error limit threshold value is 12° C for both steps 214 and 216. Depending on the system, the range of the extreme residual error limit threshold value may be between 7° C and 15° C for both steps 214 and 216. If these conditions are met, the processor 122 reports an error code for the test sensor 104 (208). In this example, the extreme residual error limit threshold value is slightly wider than residual error limit threshold value since the estimated temperature may be slightly less reliable in extreme conditions. However, the same value may be used for both of these thresholds in some examples.

If the above conditions are not met, the processor 122 determines whether: a) the measured temperature is less than the lowest temperature of the temperature range and the estimated temperature is greater or equal to a low adjusted temperature value; or b) whether the measured temperature is greater than the highest temperature of the room temperature range and the estimated temperature is less than or equal to a high adjusted temperature value (218). This step determines whether the temperature measured by the thermistor is at an extreme value while the estimated temperature is at room temperature, a combination that is consistent with a disequilibrated meter recently brought inside from a hot or cold environment. When the meter is disequilibrated, a small amount of heat is transferred to or from the sensor, causing the expected temperature of a sensor tested in a hot meter to shift up and the expected temperature of a sensor tested in a cold meter to shift down. In this example, the high and low adjusted temperature values are 2.5° C more than the high and low temperatures of the room temperature range, thus the low adjusted temperature value is 15° C and the high adjusted temperature value is 30° C. If these conditions are not met, the processor 122 uses the temperature from the temperature sensor 126 for the temperature input to the analyte concentration estimation algorithm 130 (220).

If the conditions in step 218 are met, the processor 122 compares the absolute difference between the measured temperature and the estimated temperature with an equilibrium threshold value (222). In this example, the equilibrium threshold value is 6° C. The example 6° C threshold equilibrium value can be adjusted depending on the accuracy of the temperature estimation algorithm and the desired balance of risk between a false negative and a false positive result. The threshold equilibrium value may be between 3° C and 10° C. If the difference is greater than the equilibrium threshold value, the processor 122 uses the estimated temperature for the temperature input to the analyte concentration estimation algorithm 130 (224). If the absolute difference is less than the equilibrium threshold value, the processor 122 uses the temperature from the temperature sensor 126 for the temperature input to the analyte concentration estimation algorithm 130 (222).

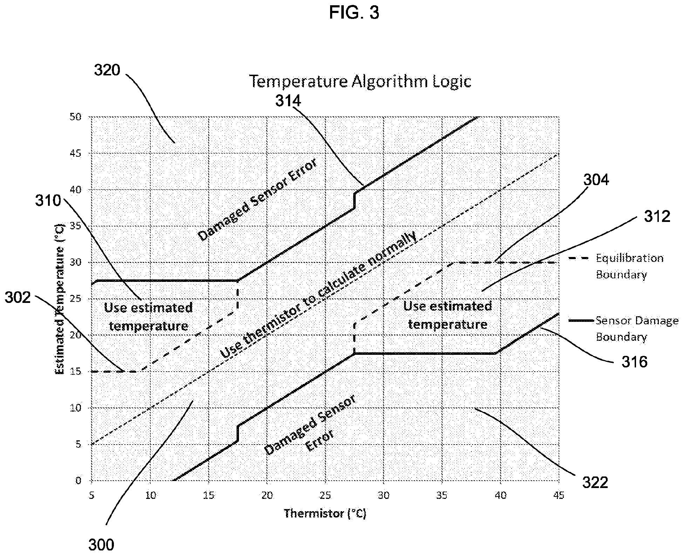

FIG. 3 is a graph showing the correlation between the estimated temperature and the measured temperature from the temperature sensor and different logic states as a result. A first area 300 represents the situations where the measured temperature is used for the temperature input to the analyte concentration estimation algorithm 130. Two areas 310 and 312 represent the situations where the estimated temperature is used for the temperature input to the analyte concentration estimation algorithm 130. The areas 310 and 312 are bounded by lines 302 and 304 that represent the boundaries of the room temperature range. The areas 310 and 312 are further bounded by lines 314 and 316 that represent the lower bounds of the equilibrium boundary. Two additional areas 320 and 322 represent situations where an error message is returned to indicate the temperature sensor 126 has been damaged.

In this example, the estimation of the temperature is determined by the temperature estimation algorithm 134. The temperature estimation algorithm 134 is derived from multiple regression analysis of input variables. Such an algorithm may be developed by performing the multiple regression based on different parameters for a specific test sensor as well as other measured signals. In this example, a multiple regression equation was developed that accurately estimates the ambient temperature using electrical signals generated from the test sensor during a glucose test (1.5°C standard deviation).

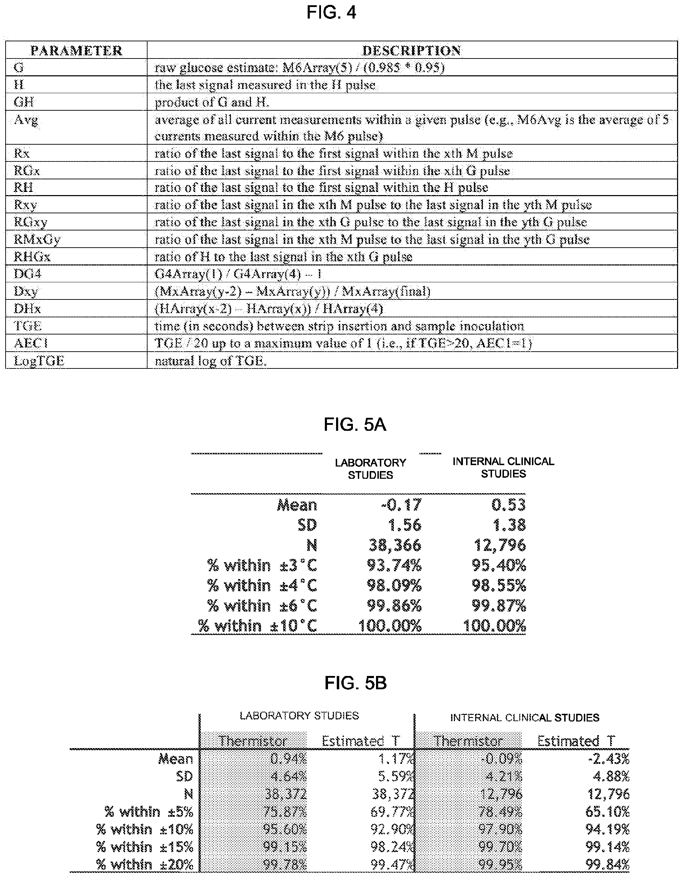

A set of training data taken from the large database of current profiles from properly equilibrated meters tested in a wide range of conditions was used to develop an equation with different terms from the parameters shown in the table in FIG. 4. The accuracy of this temperature estimation algorithm was assessed with a set of 38,367 laboratory study readings and 12,796 internal clinical study readings obtained from normally filled sensors tested with equilibrated meters. Summary statistics of the comparison between the temperature measured by a temperature sensor and the output of the example temperature estimation algorithm are included in the table shown in FIG. 5A. FIG. 5B shows a table of summary statistics for the percent error of glucose results calculated with the thermistor and with the estimated temperature using equilibrated meters. Though the temperature estimation algorithm is accurate, using this estimate when the meter is equilibrated and the thermistor is correct would cause performance to become slightly worse.

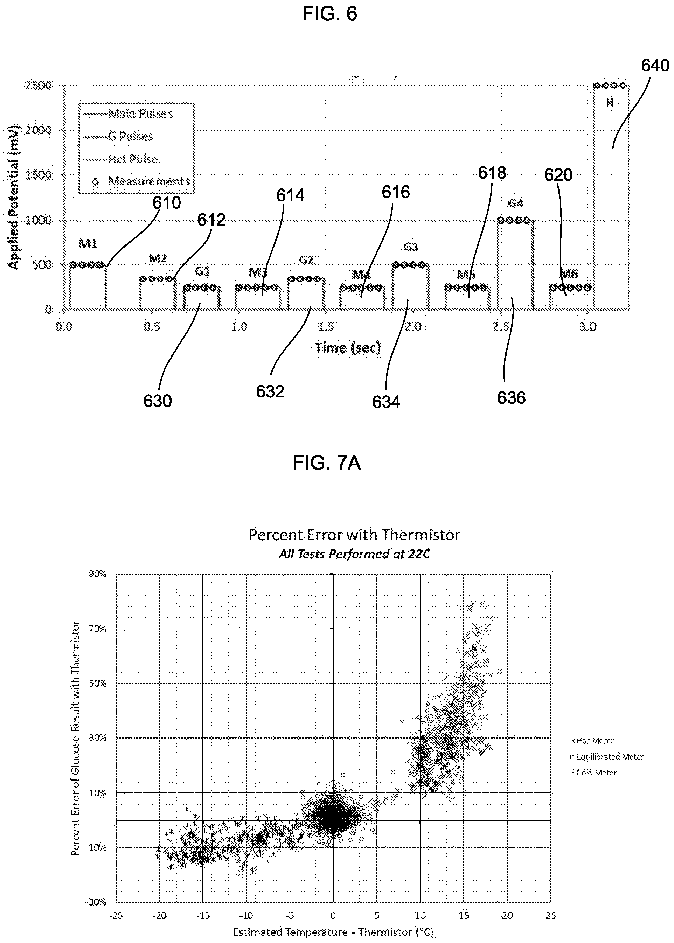

In this example, the equation for estimating temperature (in °C) includes multiple terms based on the parameters in FIG. 4 and a constant. The temperature estimate is calculated as the sum of the terms and the constant. Signals are measured during six potential pulses at the main glucose working electrode (M pulses) and four potential pulses at a bare "G" electrode in the front of the strip test chamber (G pulses). At the end of the test, a single high potential pulse is applied to the G electrode to measure signals correlated with hematocrit (H pulse). This potential input signal sequence pattern is illustrated in FIG. 6. FIG. 6 shows a series of six main pulses 610, 612, 614, 616, 618 and 620. FIG. 6 shows four pulses 630, 632, 634, and 636 at the bare G electrode. FIG. 6 also shows an input signal 640 to measure signals correlated with the hematocrit.

Current signals measured during one of the six M pulses are designated as MxArray(y), where x is the pulse number (1 - 6) and y is the measurement number within the pulse. The same convention is used for the four G pulses (i.e., GxArray(y)). Four signals are measured during the single H pulse: HArray(y). The parameters are listed in the table shown in FIG 4. Each term in the estimation equation is the product of a coefficient and an index parameter constructed from one or more measured current values.

Of course other procedures to determine estimations of temperature may be used such as by artificial neural networks with appropriate machine learning algorithms. In this example, the temperature estimation algorithm 134 provides accurate results in studies representing a wide range of temperatures, glucose concentration, and hematocrit contents.

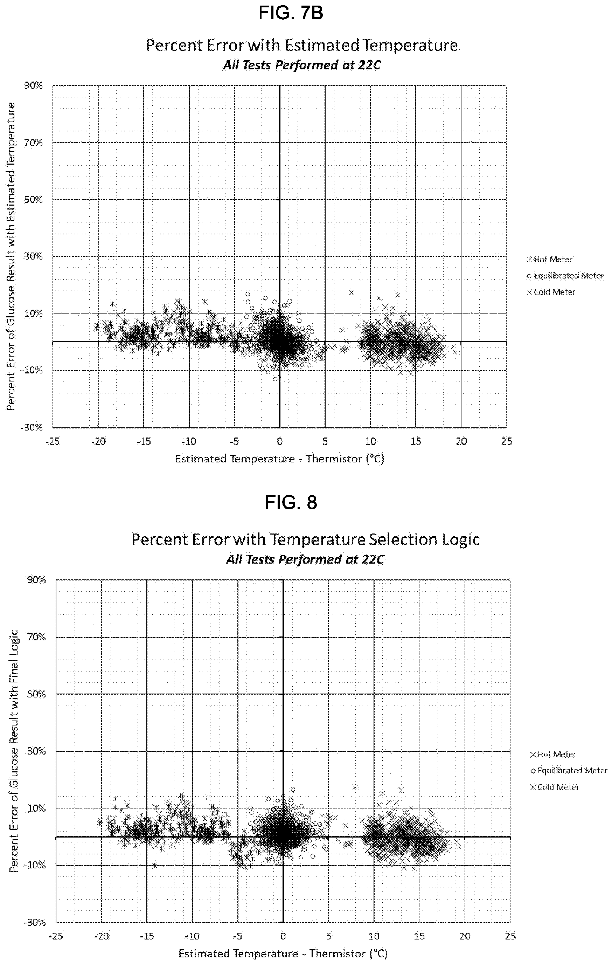

Three studies were conducted with meters stored at cold or warm temperatures and then tested at room temperature (~22°C). For the disequilibrated meters, results calculated with the incorrect thermistor values were inaccurate, particularly when the meter was colder than the test environment. Results calculated with the estimated temperature, however, were accurate in all cases. Successfully identifying when the meter is disequilibrated and then using the estimated temperature instead of the measured temperature to calculate glucose is therefore highly desirable. FIG. 7A is a graph of the plots of the error of the outputs of the analyte concentration estimation algorithm using measured temperatures from the tests conducted with meters in a wide range of equilibration states. FIG. 7B is a graph of the plots of the error of the outputs of the analyte concentration estimation algorithm using estimated temperatures from the tests conducted with meters in a wide range of equilibration states. FIG. 7C is a graph of the plots of the error of the outputs of the analyte concentration estimation algorithm using the final selected temperature (either estimated temperature or measured temperature) from the tests conducted with meters in a wide range of equilibration states. In FIGs. 7A-7B and 8, the "*" symbol represents outputs from hot meters, the "o" symbol represents outputs from equilibrated meters, and the "x" symbols represents outputs from cold meters.

FIGs. 7A-7B show that when meters are actually disequilibrated, the glucose results calculated with the estimated temperature are much more accurate than glucose results calculated with the thermistor. FIG. 8 shows that the algorithm logic worked well and correctly switched to the estimated temperature when severe disequilibration occurred, preventing the severely inaccurate results seen in FIG. 7A.

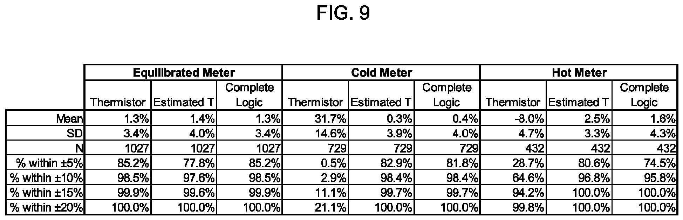

Applying the disequilibration logic produces a dramatic improvement in performance for meters that have not been allowed to equilibrate after being brought from a cold or hot environment while maintaining performance with equilibrated meters. FIG. 9 shows a table of summary data from results of studies for equilibrated meters, cold meters and hot meters in relation to the measured temperature, the estimated temperature from the example estimated temperature algorithm, and the temperature selection routine 132 in FIGs. 2A-2B.

As explained above, in addition to disequilibrated meters, the temperature selection routine 132 can determine damaged test sensors and therefore avoid using data from a damaged test sensor for an analyte concentration. Many studies have been conducted with damaged or perturbed test sensors. Such damaged test sensors produce abnormal current signals, which can affect the accuracy of the temperature estimate. The temperature selection routine 132 therefore determines if a discrepancy between the estimated and measured temperature is greater than a threshold level and that it is likely due to error in the estimated temperature rather than the measured temperature, indicating an error from the test sensor. Thus, the discrepancy is used as an error detection tool for malfunctions of the test sensor.

This logic dramatically improves the performance of meters that are actually not equilibrated while also improving the error detection success rate when test sensor damage has occurred and maintaining current performance when the test sensor is normal.

As used in this application, the terms "component," "module," "system," or the like, generally refer to a computer-related entity, either hardware (e.g., a circuit), a combination of hardware and software, software, or an entity related to an operational machine with one or more specific functionalities. For example, a component may be, but is not limited to being, a process running on a processor (e.g., digital signal processor), a processor, an object, an executable, a thread of execution, a program, and/or a computer. By way of illustration, both an application running on a controller, as well as the controller, can be a component. One or more components may reside within a process and/or thread of execution and a component may be localized on one computer and/or distributed between two or more computers. Further, a "device" can come in the form of specially designed hardware; generalized hardware made specialized by the execution of software thereon that enables the hardware to perform specific function; software stored on a computer-readable medium; or a combination thereof.

## Claims

1. An analyte concentration sensor system (100) for measuring an analyte of a fluid sample of a user, the sensor system (100) comprising:
a biosensor interface (118) operable to be connected to a test sensor (104) holding the fluid sample;
a thermistor based temperature sensor (126) configured to measure ambient temperature;
a controller coupled to the biosensor interface (118) and the temperature sensor (126), the controller being configured to:
generate an input signal to the biosensor interface (118);
read an output signal from the biosensor interface (118);
determine a measured ambient temperature from the temperature sensor (126);
execute a temperature estimation algorithm to determine an estimated ambient temperature;
determine an absolute difference between the estimated ambient temperature and the measured ambient temperature;
select one of the estimated ambient temperature or the measured ambient temperature, the selection based on the estimated ambient temperature, the measured ambient temperature, and the absolute difference between the estimated ambient temperature and the measured ambient temperature when the absolute difference is less than a largest allowed temperature compensation value;
provide the selected estimated ambient temperature or measured ambient temperature as a temperature input to an analyte concentration determination algorithm;
determine an analyte concentration from executing the analyte concentration determination algorithm; and
report an error code for the test sensor (104) if the absolute value of the difference between the estimated ambient temperature and the measured ambient temperature is greater than the largest allowed temperature compensation value.

2. The sensor system (100) of claim 1, wherein the analyte is glucose and the fluid sample is blood.

3. The sensor system (100) of claim 1, wherein the input signal is a gated amperometry pulse.

4. The sensor system (100) of claim 1, wherein the temperature estimation algorithm includes input variables determined by multiple regression analysis.

5. The sensor system (100) of claim 1, wherein the measured temperature is selected if the estimated temperature and the measured temperature are within a room temperature range.

6. The sensor system (100) of claim 5, wherein the room temperature range includes a high temperature between 25° C and 30° C and a low temperature between 13° C and 20° C.

7. The sensor system (100) of claim 1, wherein the estimated temperature is selected if the measured temperature is outside a room temperature range, but the estimated temperature is less than an adjusted high temperature of the room temperature range and greater than an adjusted low temperature of the room temperature range and the absolute value of the difference between the estimated and measured temperatures is greater than an equilibrium threshold value.

8. The sensor system (100) of claim 7, wherein the room temperature range includes a high temperature between 25° C and 30° C and a low temperature between 13° C and 20° C, and wherein the adjusted high temperature is between 27° C and 34° C and the adjusted low temperature is between 11° C and 18° C and the equilibrium threshold value is between 3° C and 10° C.

9. The sensor system (100) of claim 1, wherein, when the absolute difference between the estimated ambient temperature and the measured ambient temperature is less than the largest allowed temperature compensation value, the controller is configured to report an error code for the test sensor (104) if the measured temperature and the estimated temperature are outside a room temperature range in opposite directions.

10. The sensor system (100) of claim 1, wherein, when the absolute difference between the estimated ambient temperature and the measured ambient temperature is less than the largest allowed temperature compensation value, the controller is Z configured to report an error code for the test sensor (104) if the measured temperature is within a room temperature range and the difference between the estimated temperature and the measured temperature is greater than a residual error limit threshold value.

11. The sensor system (100) of claim 10, wherein the controller is configured to report an error code for the test sensor (104) if the measured temperature and estimated temperature are higher than the highest temperature of the room temperature range and the difference between the estimated temperature and the measured temperature is greater than an extreme residual error limit threshold value.

12. The sensor system (100) of claim 10, wherein the controller is configured to report an error code for the test sensor (104) if the measured temperature and estimated temperature are lower than the lowest temperature of the room temperature range and the difference between the measured temperature and the estimated temperature is greater than an extreme residual error limit threshold value.

13. A method to determine suitability of an ambient temperature measurement from a thermistor temperature sensor in an analyte meter, the analyte meter including a biosensor interface (118) operable to be connected to a test sensor (104) holding a fluid sample, and a controller, the method comprising:
generating an input signal to the interface when connected to the test sensor (104) with the fluid sample;
determining an output signal from the test sensor (104);
determining a measured ambient temperature from a thermistor based temperature sensor (126);
determining an estimated ambient temperature from a temperature estimation algorithm via the controller;
determining an absolute difference between the estimated ambient temperature and the measured ambient temperature via the controller;
selecting one of the estimated ambient temperature or the measured ambient temperature via the controller based on the estimated ambient temperature, the ambient measured temperature, and the absolute difference between the estimated ambient temperature and the measured ambient temperature when the absolute difference is less than a largest allowed temperature compensation value;
providing the selected estimated ambient temperature or measured ambient temperature as a temperature input to an analyte concentration determination algorithm;
determining an analyte concentration from executing the analyte concentration determination algorithm; and
reporting an error code for the test sensor (104) if the absolute value of the difference between the estimated ambient temperature and the measured ambient temperature is greater than the largest allowed temperature compensation value.

## Patentansprüche

1. Ein Analytkonzentrationssensorsystem (100) zum Messen eines Analyten in einer Fluidprobe eines Benutzers, das Sensorsystem (100) umfassend:
eine Biosensorschnittstelle (118), die so betreibbar ist, dass sie mit einem Testsensor (104) verbunden werden kann, der die Fluidprobe enthält;
einen thermistorbasierten Temperatursensor (126), der dazu eingerichtet ist, eine Umgebungstemperatur zu messen;
eine Steuerung, die mit der Biosensorschnittstelle (118) und dem Temperatursensor (126) gekoppelt ist, wobei die Steuerung dazu eingerichtet ist,
ein Eingangssignal für die Biosensorschnittstelle (118) zu erzeugen;
ein Ausgangssignal von der Biosensorschnittstelle (118) zu lesen;
eine gemessene Umgebungstemperatur von dem Temperatursensor (126) zu bestimmen;
einen Temperaturschätzalgorithmus auszuführen, um eine geschätzte Umgebungstemperatur zu bestimmen;
eine absolute Differenz zwischen der geschätzten Umgebungstemperatur und der gemessenen Umgebungstemperatur zu bestimmen;
die geschätzte Umgebungstemperatur oder die gemessene Umgebungstemperatur auszuwählen, wobei die Auswahl auf der geschätzten Umgebungstemperatur, der gemessenen Umgebungstemperatur und der absoluten Differenz zwischen der geschätzten Umgebungstemperatur und der gemessenen Umgebungstemperatur basiert, wenn die absolute Differenz kleiner als ein größter zulässiger Temperaturkompensationswert ist;
die ausgewählte geschätzte Umgebungstemperatur oder gemessene Umgebungstemperatur als eine Temperatureingabe für einen Analytkonzentrations-Bestimmungsalgorithmus bereitzustellen;
eine Analytkonzentration aus dem Ausführen des Analytkonzentrations-Bestimmungsalgorithmus zu bestimmen; und
einen Fehlercode für den Testsensor (104) zu melden, wenn der Absolutwert der Differenz zwischen der geschätzten Umgebungstemperatur und der gemessenen Umgebungstemperatur größer ist als der größte zulässige Temperaturkompensationswert.

2. Das Sensorsystem (100) nach Anspruch 1, wobei der Analyt Glukose ist und die Fluidprobe Blut ist.

3. Das Sensorsystem (100) nach Anspruch 1, wobei das Eingangssignal ein Puls-Amperometrieimpuls ist.

4. Das Sensorsystem (100) nach Anspruch 1, wobei der Temperaturschätzalgorithmus durch multiple Regressionsanalyse bestimmte Eingabevariablen enthält.

5. Das Sensorsystem (100) nach Anspruch 1, wobei die gemessene Temperatur ausgewählt wird, wenn die geschätzte Temperatur und die gemessene Temperatur innerhalb eines Raumtemperaturbereichs liegen.

6. Das Sensorsystem (100) nach Anspruch 5, wobei der Raumtemperaturbereich eine hohe Temperatur zwischen 25° C und 30° C und eine niedrige Temperatur zwischen 13° C und 20° C enthält.

7. Das Sensorsystem (100) nach Anspruch 1, wobei die geschätzte Temperatur ausgewählt wird, wenn die gemessene Temperatur außerhalb eines Raumtemperaturbereichs liegt, aber die geschätzte Temperatur kleiner als eine eingestellte hohe Temperatur des Raumtemperaturbereichs und größer als eine eingestellte niedrige Temperatur des Raumtemperaturbereichs ist und der Absolutwert der Differenz zwischen der geschätzten und der gemessenen Temperatur größer als ein Gleichgewichtsschwellenwert ist.

8. Das Sensorsystem (100) nach Anspruch 7, wobei der Raumtemperaturbereich eine hohe Temperatur zwischen 25° C und 30° C und eine niedrige Temperatur zwischen 13° C und 20° C enthält, und wobei die eingestellte hohe Temperatur zwischen 27° C und 34° C und die eingestellte niedrige Temperatur zwischen 11° C und 18° C liegt und der Gleichgewichtsschwellenwert zwischen 3° C und 10° C liegt.

9. Das Sensorsystem (100) nach Anspruch 1, wobei, wenn die absolute Differenz zwischen der geschätzten Umgebungstemperatur und der gemessenen Umgebungstemperatur kleiner ist als der größte zulässige Temperaturkompensationswert, die Steuerung dazu eingerichtet ist, einen Fehlercode für den Testsensor (104) zu melden, wenn die gemessene Temperatur und die geschätzte Temperatur in entgegengesetzten Richtungen außerhalb eines Raumtemperaturbereichs liegen.

10. Das Sensorsystem (100) nach Anspruch 1, wobei, wenn die absolute Differenz zwischen der geschätzten Umgebungstemperatur und der gemessenen Umgebungstemperatur kleiner ist als der größte zulässige Temperaturkompensationswert, die Steuerung dazu eingerichtet ist, einen Fehlercode für den Testsensor (104) zu melden, wenn die gemessene Temperatur innerhalb eines Raumtemperaturbereichs liegt und die Differenz zwischen der geschätzten Temperatur und der gemessenen Temperatur größer ist als ein Restfehlergrenzwert.

11. Das Sensorsystem (100) nach Anspruch 10, wobei die Steuerung dazu eingerichtet ist, einen Fehlercode für den Testsensor (104) zu melden, wenn die gemessene Temperatur und die geschätzte Temperatur höher sind als die höchste Temperatur des Raumtemperaturbereichs und die Differenz zwischen der geschätzten Temperatur und der gemessenen Temperatur größer ist als ein extremer Restfehlergrenzwert.

12. Das Sensorsystem (100) nach Anspruch 10, wobei die Steuerung dazu eingerichtet ist, einen Fehlercode für den Testsensor (104) zu melden, wenn die gemessene Temperatur und die geschätzte Temperatur niedriger sind als die niedrigste Temperatur des Raumtemperaturbereichs und die Differenz zwischen der gemessenen Temperatur und der geschätzten Temperatur größer ist als ein extremer Restfehlergrenzwert.

13. Ein Verfahren zum Bestimmen einer Eignung einer Umgebungstemperaturmessung von einem Thermistor-Temperatursensor in einem Analysemessgerät, wobei das Analysemessgerät eine Biosensor-Schnittstelle (118), die so betreibbar ist, dass sie mit einem Testsensor (104) verbunden werden kann, der eine Fluidprobe enthält, und eine Steuerung enthält, das Verfahren umfassend:
Erzeugen eines Eingangssignals für die Schnittstelle, wenn sie mit dem Testsensor (104) mit der Fluid-Probe verbunden ist;
Bestimmen eines Ausgangssignals von dem Prüfsensor (104);
Bestimmen einer gemessenen Umgebungstemperatur von einem thermistorbasierten Temperatursensor (126);
Bestimmen einer geschätzten Umgebungstemperatur aus einem Temperaturschätzalgorithmus über die Steuerung;
Bestimmen einer absoluten Differenz zwischen der geschätzten Umgebungstemperatur und der gemessenen Umgebungstemperatur über die Steuerung;
Auswählen der geschätzten Umgebungstemperatur oder der gemessenen Umgebungstemperatur über die Steuerung basierend auf der geschätzten Umgebungstemperatur, der gemessenen Umgebungstemperatur und der absoluten Differenz zwischen der geschätzten Umgebungstemperatur und der gemessenen Umgebungstemperatur, wenn die absolute Differenz kleiner als ein größter zulässiger Temperaturkompensationswert ist;
Bereitstellen der ausgewählten geschätzten Umgebungstemperatur oder gemessenen Umgebungstemperatur als Temperatureingabe für einen Analytkonzentrations-Bestimmungsalgorithmus;
Bestimmen einer Analytkonzentration durch Ausführen des Analytkonzentrations-Bestimmungsalgorithmus; und
Melden eines Fehlercodes für den Testsensor (104), wenn der Absolutwert der Differenz zwischen der geschätzten Umgebungstemperatur und der gemessenen Umgebungstemperatur größer ist als der größte zulässige Temperaturkompensationswert.

## Revendications

1. Système de capteur de concentration d'analyte (100) pour mesurer un analyte d'un échantillon de fluide d'un utilisateur, le système de capteur (100) comprenant :
une interface de biocapteur (118) pouvant être connectée à un capteur de test (104) contenant l'échantillon de liquide ;
un capteur de température à thermistance (126) configuré pour mesurer la température ambiante ;
un contrôleur couplé à l'interface de biocapteur (118) et au capteur de température (126), le contrôleur étant configuré pour :
générer un signal d'entrée vers l'interface de biocapteur (118) ;
lire un signal de sortie de l'interface de biocapteur (118) ;
déterminer une température ambiante mesurée à partir du capteur de température (126) ;
exécuter un algorithme d'estimation de la température pour déterminer une température ambiante estimée ;
déterminer une différence absolue entre la température ambiante estimée et la température ambiante mesurée ;
sélectionner l'une de la température ambiante estimée ou de la température ambiante mesurée, la sélection basée sur la température ambiante estimée, la température ambiante mesurée et la différence absolue entre la température ambiante estimée et la température ambiante mesurée lorsque la différence absolue est inférieure à une valeur de compensation de température autorisée la plus grande ;
fournir la température ambiante estimée ou la température ambiante mesurée sélectionnée comme entrée de température à un algorithme de détermination de la concentration d'analyte ;
déterminer une concentration d'analyte à partir de l'exécution de l'algorithme de détermination de la concentration d'analyte ; et
signaler un code d'erreur pour le capteur de test (104) si la valeur absolue de la différence entre la température ambiante estimée et la température ambiante mesurée est supérieure à la plus grande valeur de compensation de température autorisée.

2. Le système de capteur (100) de la revendication 1, dans lequel l'analyte est le glucose et l'échantillon de fluide est le sang.

3. Le système de capteur (100) de la revendication 1, dans lequel le signal d'entrée est une impulsion d'ampérométrie à porte.

4. Le système de capteur (100) de la revendication 1, dans lequel l'algorithme d'estimation de la température comprend des variables d'entrée déterminées par analyse de régression multiple.

5. Le système de capteur (100) de la revendication 1, dans lequel la température mesurée est sélectionnée si la température estimée et la température mesurée se situent dans une plage de température ambiante.

6. Le système de capteur (100) de la revendication 5, dans lequel la plage de température ambiante comprend une température élevée comprise entre 25° C et 30° C et une température basse comprise entre 13° C et 20° C.

7. Le système de capteur (100) de la revendication 1, dans lequel la température estimée est sélectionnée si la température mesurée est en dehors d'une plage de température ambiante, mais que la température estimée est inférieure à une température élevée ajustée de la plage de température ambiante et supérieure à une température basse ajustée de la plage de température ambiante, et que la valeur absolue de la différence entre les températures estimée et mesurée est supérieure à une valeur seuil d'équilibre.

8. Le système de capteur (100) de la revendication 7, dans lequel la plage de température ambiante comprend une température élevée comprise entre 25° C et 30° C et une température basse comprise entre 13° C et 20° C, et dans lequel la température élevée ajustée est comprise entre 27° C et 34° C et la température basse ajustée est comprise entre 11° C et 18° C et la valeur seuil d'équilibre est comprise entre 3° C et 10° C.

9. Le système de capteur (100) de la revendication 1, 2 dans lequel, lorsque la différence absolue entre la température ambiante estimée et la température ambiante mesurée est inférieure à la plus grande valeur de compensation de température autorisée, le contrôleur est configuré pour signaler un code d'erreur pour le capteur de test (104) si la température mesurée et la température estimée sont en dehors d'une plage de température ambiante dans des directions opposées.

10. Le système de capteur (100) de la revendication 1, 2 dans lequel, lorsque la différence absolue entre la température ambiante estimée et la température ambiante mesurée est inférieure à la plus grande valeur de compensation de température autorisée, le contrôleur est configuré pour signaler un code d'erreur pour le capteur de test (104) si la température mesurée est dans une plage de température ambiante et que la différence entre la température estimée et la température mesurée est supérieure à une valeur seuil de limite d'erreur résiduelle.

11. Le système de capteur (100) de la revendication 10, dans lequel le contrôleur est configuré pour signaler un code d'erreur pour le capteur de test (104) si la température mesurée et la température estimée sont supérieures à la température la plus élevée de la plage de température ambiante et si la différence entre la température estimée et la température mesurée est supérieure à une valeur seuil de limite d'erreur résiduelle extrême.

12. Le système de capteur (100) de la revendication 10, dans lequel le contrôleur est configuré pour signaler un code d'erreur pour le capteur de test (104) si la température mesurée et la température estimée sont inférieures à la température la plus basse de la plage de température ambiante et que la différence entre la température mesurée et la température estimée est supérieure à une valeur seuil de limite d'erreur résiduelle extrême.

13. Méthode pour déterminer l'aptitude d'une mesure de la température ambiante à partir d'un capteur de température à thermistance dans un analyseur, l'analyseur comprenant une interface de biocapteur (118) pouvant être connectée à un capteur de test (104) contenant un échantillon de fluide, et un contrôleur, la méthode comprenant
générer un signal d'entrée vers l'interface lorsque connecté au capteur de test (104) avec l'échantillon de fluide ;
déterminer un signal de sortie à partir du capteur de test (104) ;
déterminer une température ambiante mesurée à partir d'un capteur de température à thermistance (126) :
déterminer une température ambiante estimée à partir d'un algorithme d'estimation de la température via le contrôleur ;
déterminer une différence absolue entre la température ambiante estimée et la température ambiante mesurée via le contrôleur ;
sélectionner l'une de la température ambiante estimée ou de la température ambiante mesurée via le contrôleur en fonction de la température ambiante estimée, de la température ambiante mesurée et de la différence absolue entre la température ambiante estimée et la température ambiante mesurée lorsque la différence absolue est inférieure à une valeur de compensation de température autorisée la plus grande
fournir la température ambiante estimée ou la température ambiante mesurée sélectionnée comme une entrée de température à un algorithme de détermination de la concentration d'analyte ;
déterminer une concentration d'analyte à partir de l'exécution de l'algorithme de détermination de la concentration d'analyte ; et
signaler un code d'erreur pour le capteur de test (104) si la valeur absolue de la différence entre la température ambiante estimée et la température ambiante mesurée est supérieure à la plus grande valeur de compensation de température autorisée.
